# EUROPEAN PATENT APPLICATION

(11) **EP 2 848 242 A1**
(43) Date of publication of application: **18.03.2015**
(21) Application number: 14184491.0
(22) Date of filing: 11.09.2014
(51) Int. Cl.: A61K 9/00, A61K 9/20, A61K 31/155

(54) **Orally disintegrating formulations of Linagliptin**

(30) Priority: 12.09.2013 TR 201310724; 24.09.2013 TR 201311200
(71) Applicant: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Saydam, Mehtap, 34460 Istanbul (TR)
(74) Representative: Sevinç, Erkan

(57) **Abstract**

The present invention relates to orally disintegrating formulations comprising linagliptin or a pharmaceutically acceptable salt thereof and one or more pharmaceutically acceptable excipients.

## Description

### Field of Invention

The present invention relates to an orally disintegrating formulation comprising linagliptin or a pharmaceutically acceptable salt thereof and one or more pharmaceutically acceptable excipients.

### Background of Invention

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

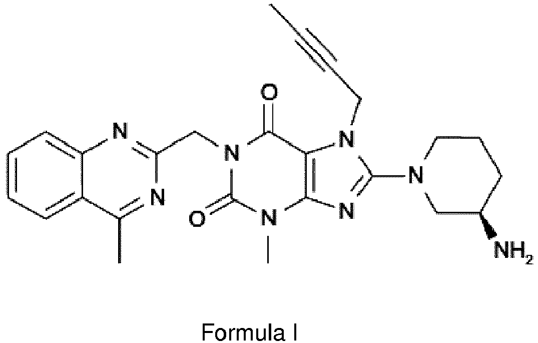

Its chemical name is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

There is Linagliptin coated tablet formulation license in the US and EU under the brand name TRADJENTA^{®} by Boehringer Ingelhem International. TRADJENTA^{®} includes mannitol, prejelatinized starch, maize starch, copovidone, magnesium stearate in the tablet core and hypromellose, titanium dioxide, talc, macrogol 6000 and red iron oxide in the film coating.

It is used once daily and can be used either alone or in combination with insulin or other glycemic agents.

EP 2 023 902 B1 provides pharmaceutical formulations comprising DPP-4 inhibitors included Linagliptin. It is indicated that DPP-4 inhibitors with primary amine group shows incompatibilities, degradation problems or extraction problems with excipients such as microcrystalline cellulose, sodium starch glycolate, croscarmellose sodium, tartaric acid, citric acid, glucose, fructose, saccharose, lactose, maltodextrines. Linagliptin has also a primary amine group on its chemical structure. In solid dosage forms, it may react with many excipients or impurities of excipients, although linagliptin itself is very stable. Thus, aforementioned patent provides Linagliptin tablet formulations which do not comprise above excipients.

In the state of art, there are several patents and applications which disclose tablet formulations of linagliptin but none of them comprises orally disintegrating formulations of linagliptin.

Over the past decades, orally disintegrating tablets (ODTs) have gained considerable attention as a preferred alternative to conventional tablets and capsules due to better patient compliance. There are a growing number of people who cannot swallow tablets or capsules. ODTs are solid dosage forms which are not needed to swallow and disintegrate rapidly in mouth through the saliva. They have also advantageous for administrations of medicaments to patients who are traveling or have little access to water or patients who are mentally retarded, uncooperative or nauseated. Furthermore, with ODT tablets rapid drug therapy intervention and increased bioavailability of drugs are possible. Because the pre-gastric drug absorption avoids the first-pass metabolism, the drug dose can be reduced if a significant amount of the drug is lost through the hepatic metabolism.

On the other side, it is difficult to develop orally disintegrating formulations because of several different requirements such as fast disintegration and stability. ODT must disintegrate in the oral cavity with the existence of saliva in a short period of time and so, those compositions should have a porous structure. However, these porous characteristic tend to be very sensitive to humidity and it may lead to process related problems, stability and friability problems during packaging and transportation.

To fulfill all these requirements, orally disintegrating formulations comprising linagliptin or a pharmaceutically acceptable salt thereof and one or more pharmaceutically acceptable excipients have been developed. To achieve desired disintegration time and ensure the stability and suitable friability of formulation, sodium starch glycolate and microcrystalline cellulose has been used as suitable excipients. In addition, to further improve the process, sodium stearyl fumarate has been selected as a lubricant.

### Description of the invention

The main embodiment of the present invention is to provide an orally disintegrating formulation comprising linagliptin or a pharmaceutically acceptable salt thereof and one or more pharmaceutically acceptable excipients.

According to one embodiment, linagliptin or a pharmaceutically acceptable salt thereof is present in an amount of 2 to 80 %, preferably 3.5 to 60 % and more preferably 10 to 40 % by weight of total formulation.

According to another embodiment, the formulation of linagliptin is disintegrated in less than 1 min.

The orally disintegrating formulation of this invention comprising one or more pharmaceutically acceptable excipient is selected from the group comprising super disintegrants, diluents, lubricants, binders, sweeteners, aroma, acidifying agents and alkalizing agents.

An object of the present invention is to obtain an orally disintegrating formulation of linagliptin which provides desired disintegration time and avoids the stability, friability and process related problems.

As used herein, the term "super-disintegrant" is defined as the pharmaceutical ingredient that provides disintegration substantially faster than the conventional disintegrants. According to this embodiment, the super-disintegrant is selected from the group comprising sodium starch glycolate, croscarmellose sodium, sodium carboxymethyl starch, soy polysaccharide, cross-linked alginic acid, crospovidone, gellan gum, xanthan gum, calcium silicate or ion exchange resins, preferably sodium starch glycolate.

In this invention, desired disintegration time has been achieved with sodium starch glycolate. It rapidly swells in saliva and leads faster disintegration than the other super-disintegrants. Moreover, it is surprisingly found that tablet compression force does not affect the disintegration time of formulation comprising linagliptin.

In this embodiment, the amount of sodium starch glycolate is in the range of 0.1 to 50 %, preferably 0.25 to 25 %, more preferably it is 0.25 to 10 % by weight of total formulation.

In another embodiment, the diluent is selected from the group comprising microcrystalline cellulose, inorganic salts, polysaccharides, dextrose, dicalsium phosphate, dextrates, lactitol, trehalose, heavy magnesium carbonate, dibasic calcium phosphate, tribasic calcium phosphate, sorbitol, sucrose, trehalose, isomalt, mannitol, lactose, starch, sodium carbonate, sodium bicarbonate, sodium chloride, maltodextrine, calcium carbonate, xylitol or mixtures thereof, preferably microcrystalline cellulose.

According to one embodiment, microcrystalline cellulose is a diluent which has the best flowability properties among the other diluents. In this invention, it further improves the disintegration of formulation as well as being a diluent. In addition, it further enhances the compressibility by increasing the hardness of the tablet.

In this embodiment, the amount of microcrystalline cellulose is in the range of 0.5 to 90 %, preferably 0.5 to 60 %, more preferably it is 0.5 to 30 % by weight of total formulation.

In another embodiment, ODT tablets are sensitive formulations that are difficult to store and transport. In this invention, we have achieved desired friability as well as desired disintegration time by using both starch glycolate and microcrystalline cellulose in a specific ratio in linagliptin formulation. Thus, during transportation and packaging, any deformation has not been observed.

Friability test has been performed by using a drum with an internal diameter between 283-291 mm and a depth between 36-40 mm.

According to this embodiment, the ratio of that **microcrystalline cellulose to sodium starch glycolate** is in the range of 0.1 to 30 (w/w), preferably 0.1 to 20 (w/w) and more preferably it is 0.1 to 10 (w/w).

According to another embodiment, the lubricant is selected from the group comprising sodium stearyl fumarate, magnesium stearate, polyethylene glycol (PEG), sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably sodium stearyl fumarate.

In general, hydrophobic properties of magnesium stearate as a lubricant increase the process time with prolonged mixing and cause slow disintegration. In this invention, **sodium stearyl fumarate** is selected as a suitable lubricant in tableting. Due to its hydrophilic property, formulation does not have the disadvantages of magnesium stearate in respect of process and disintegration problems.

According to this embodiment, the amount of **sodium stearyl fumarate** is in the range of 0.1 to 10%, preferably 0.1 to 5% and more preferably it is 0.25 to 3% by weight of total formulation.

Suitable binders may include but not limited to xylitol, sucrose stearate, sugars, glucose syrup, natural gums, starch, gelatin, polyvinylpyrrolidone, polymethacrylates; collagen, proteins such as gelatin; agar, alginate, sodium alginate, pectin, starch, carboxymethyl cellulose, hydroxypropyl cellulose, methyl cellulose and similar semi-synthetic polymers; carbomer, poloxamer, polyacrylamide, polyvinyl alcohol and similar synthetic polymers; aluminum hydroxide, bentonite, laponite and other inorganic substances; starch mucilage, acacia mucilage, polydextrose, polyethylene oxide, or the mixtures thereof.

Suitable fillers may include but not limited to lactose, sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, izomalt and mixtures thereof.

Suitable sweeteners may include but not limited to sucralose, thaumatin, mogroside, inuline, erythritol, or mixtures thereof.

Suitable aromas may include but not limited to fruit aromas such as orange, banana, strawberry, cherry, wild cherry, lemon, etc., and other aromas such as cardamom, anis, mint, menthol, vanillin, and the mixtures thereof.

Suitable acidifying agents may include but not limited to tartaric acid, citric acid anhydride, fumaric acid, citric acid, adipic acid, acetic acid, hydrochloric acid, malic acid, nitric acid, phosphoric acid, sulfuric acid and mixtures thereof.

Suitable alkalizing agents may include but not limited to sodium bicarbonate, sodium glycine carbonate, ammonia solution, ammonium carbonate, diethanolamine, diisopropanolamine, potassium hydroxide, sodium borate, sodium carbonate, sodium hydroxide, trolamine and mixtures thereof.

Coating may also preferably be used for moisture protection. It can be selected from the group comprising Polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer(PVP-VA), and all kinds of OpadryTM, as well as pigments, dyes, titanium dioxide and iron oxide, talk and polymethylmetacrylate copolymers (Eudragit).

In this present invention, to achieve desired disintegration time and friability with stability and an improved process, this formulation has been designed, comprising the following:
a. 2.0 - 80.0 % by weight of linagliptin
b. 0.1 - 50 % by weight of sodium starch glycolate
c. 0.5 - 90.0 % by weight of microcrystalline cellulose
d. 0.1 - 1.0 % by weight of silica
e. 0.1 - 2.0 % by weight of sweetener
f. 0.1 - 5.0 % by weight of aroma
g. 0.1 - 10.0 % by weight of sodium stearyl fumarate

a. 2.0 - 80.0 % by weight of linagliptin
b. 0.1 - 50.0 % by weight of sodium starch glycolate
c. 0.5 - 90.0 % by weight of microcrystalline cellulose
d. 5.0 - 60.0 % by weight of tartaric acid
e. 5.0 - 60.0 % by weight of sodium bicarbonate
f. 0.1 - 2.0 % by weight of sweetener
g. 0.1 - 5.0 % by weight of aroma
h. 0.1- 10.0 % by weight of sodium stearyl fumarate and

a. 2.0 - 80.0 % by weight of linagliptin
b. 0.1 - 50.0 % by weight of sodium starch glycolate
c. 0.5 - 90.0 % by weight of microcrystalline cellulose
d. 0.1 - 1.0 % by weight of silica
e. 0.1 - 2.0 % by weight of sweetener
f. 0.1 - 5.0 % by weight of aroma
g. 0.1 - 10.0 % by weight of sodium stearyl fumarate
h. 5.0 - 60.0 % by weight of sodium bicarbonate
i. 5.0 - 60.0 % by weight of citric acid anhydrate
j. 2.5 - 5.5 % by weight of xylitol
k. 0.2 - 1.0 % by weight of sucrose stearate
l. 3.0 - 5.0 % by weight of coating

### Example 1: Direct compression

| **Ingredients** | **% (amount)** |
|---|---|
| Linagliptin | 3.5 - 60.0 |
| microcrystalline cellulose | 0.5 - 60.0 |
| sodium starch glycolate | 0.25 - 25.0 |
| Silica | 0.1 - 1.0 |
| sweetener | 0.1 - 2.0 |
| aroma | 0.1 - 5.0 |
| sodium stearyl fumarate | 0.25 - 3.0 |

The production of the formulation is carried out as follows: Linagliptin, sweetener, aroma, sodium starch glycolate and microcrystalline cellulose are mixed. Then, silica and sodium stearyl fumarate are added and mixed respectively. The mixture is pressed into tablets.

### Example 2: Dry granulation

| **Ingredients** | **% (amount)** |
|---|---|
| Linagliptin | 3.5 - 60.0 |
| microcrystalline cellulose | 0.5 - 60.0 |
| sodium starch glycolate | 0.25 - 25.0 |
| Silica | 0.1 - 1.0 |
| sweetener | 0.1 - 2.0 |
| aroma | 0.1 - 5.0 |
| sodium stearyl fumarate | 0.25 - 3.0 |

The production of the formulation is carried out as follows: Linagliptin, sweetener, aroma, sodium starch glycolate and microcrystalline cellulose, silica and part of sodium stearyl fumarate are mixed and passed through roller compactor. Granules are sieved and mixed with other part of sodium stearyl fumarate. Tablet compression is performed.

### Example 3: Fast - Melt Tablets with Direct compression

| **Ingredients** | **% (amount)** |
|---|---|
| Linagliptin | 3.5 - 60.0 |
| microcrystalline cellulose | 0.5 - 60.0 |
| sodium starch glycolate | 0.1 - 50.0 |
| tartaric acid | 5.0 - 60.0 |
| sodium bicarbonate | 5.0 - 60.0 |
| sweetener | 0.1 - 2.0 |
| aroma | 0.1 - 5.0 |
| sodium stearyl fumarate | 0.25 - 3.0 |
| Opadry AMB/ Kollicoat IR | 3.0 - 5.0 |

The production of the formulation is carried out as follows: Linagliptin, sodium starch glycolate, sodium bicarbonate and tartaric acid are mixed. Microcrystalline cellulose, sweetener and aroma is added to the powder mixture and mixed. Then, sodium stearyl fumarate sieved and added to the mixture and mixed again. Direct compression is performed with the final mixture. Pressed tablets are preferably coated with PVA (polyvinyl alcohol) based coatings such as Opadry AMB/ Kollicoat IR.

### Example 4: Fast- Melt Tablets with Hot - Melt extrusion

| **Ingredients** | **% (amount)** |
|---|---|
| Linagliptin | 3.5 - 60.0 |
| microcrystalline cellulose | 0.5 - 60.0 |
| sodium starch glycolate | 0.25 - 25.0 |
| Silica | 0.1 - 1.0 |
| sweetener | 0.1 - 2.0 |
| aroma | 0.1 - 5.0 |
| sodium stearyl fumarate | 0.25 - 3.0 |
| sodium bicarbonate | 5.0 - 60.0 |
| citric acid anhydrate | 5.0 - 60.0 |
| xylitol | 2.5 - 5.5 |
| sucrose stearate | 0.2 - 1.0 |
| Opadry AMB/ Kollicoat IR | 3.0 - 5.0 |

The production of the formulation is carried out as follows: Linagliptin, sodium bicarbonate, citric acid anhydrate, xylitol and sucrose stearate are mixed and melt in hot - melt extruder at 50-180 °C. With the help of thermal binders (xylitol and sucrose stearate), granules are obtained. Granules are sieved and microcrystalline cellulose, sodium starch glycolate, silica, aroma and sweetener are added and mixed. Then, sodium stearyl fumarate is sieved and added to this mixture and mixed. Total powder mixture is pressed into tablets. Pressed tablets are preferably coated with PVA (polyvinyl alcohol) based coatings such as Opadry AMB/ Kollicoat IR.

## Claims

1. An orally disintegrating formulation comprising linagliptin or a pharmaceutically acceptable salt thereof in an amount of 2 to 80 %, preferably 3.5 to 60 % and more preferably 10 to 40 % by weight of total formulation and one or more pharmaceutically acceptable excipients.

2. The orally disintegrating formulation according to claim 1, wherein one or more pharmaceutically acceptable excipient is selected from the group comprising super disintegrants, diluents, lubricants, binders, sweeteners, aroma, acidifying agents and alkalizing agents.

3. The orally disintegrating formulation according to claim 3, wherein the super-disintegrant is selected from the group comprising starch glycolate, croscarmellose sodium, sodium carboxymethyl starch, soy polysaccharide, cross-linked alginic acid, crospovidone, gellan gum, xanthan gum, calcium silicate or ion exchange resins, preferably sodium starch glycolate.

4. The orally disintegrating formulation according to claims 4, the amount of sodium starch glycolate is in the range of 0.1 to 50 %, preferably 0.25 to 25 %, more preferably it is 0.25 to 10 % by weight of total formulation

5. The orally disintegrating formulation according to claim 3, wherein the diluent is selected from the group comprising microcrystalline cellulose, inorganic salts, polysaccharides, dextrose, dicalsium phosphate, dextrates, lactitol, trehalose, heavy magnesium carbonate, dibasic calcium phosphate, tribasic calcium phosphate, sorbitol, sucrose, trehalose, isomalt, mannitol, lactose, starch, sodium carbonate, sodium bicarbonate, sodium chloride, maltodextrine, calcium carbonate, xylitol or mixtures thereof, preferably microcrystalline cellulose.

6. The orally disintegrating formulation according to claim 7, the amount of microcrystalline cellulose is in the range of 0.5 to 90 %, preferably 0.5 to 60 %, more preferably it is 0.5 to 30 % by weight of total formulation.

7. The orally disintegrating formulation according to any preceding claims, the ratio of that microcrystalline cellulose to sodium starch glycolate is in the range of 0.1 to 30 (w/w), preferably 0.1 to 20 (w/w) and more preferably it is 0.1 to 10 (w/w).

8. The orally disintegrating formulation according to any preceding claims, the lubricant is selected from the group comprising sodium stearyl fumarate, magnesium stearate, polyethylene glycol (PEG), sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably sodium stearyl fumarate.

9. The orally disintegrating formulation according to any preceding claims, the amount of sodium stearyl fumarate is in the range of 0.1 to 10%, preferably 0.1 to 5% and more preferably it is 0.25 to 3% by weight of total formulation.

10. The orally disintegrating formulation-according to any preceding claim comprising;
a) 2.0 - 80.0 % by weight of linagliptin
b) 0.1 - 50 % by weight of sodium starch glycolate
c) 0.5 - 90.0 % by weight of microcrystalline cellulose
d) 0.1 - 1.0 % by weight of silica
e) 0.1 - 2.0 % by weight of sweetener
f) 0.1 - 5.0 % by weight of aroma
g) 0.1 - 10.0 % by weight of sodium stearyl fumarate
h) 3.0 - 5.0 % by weight of coating

11. The orally disintegrating formulation-according to any preceding claim comprising;
a) 2.0 - 80.0 % by weight of linagliptin
b) 0. 1 - 50.0 % by weight of sodium starch glycolate
c) 0.5 - 90.0 % by weight of microcrystalline cellulose
d) 5.0 - 60.0 % by weight of tartaric acid
e) 5.0 - 60.0 % by weight of sodium bicarbonate
f) 0.1 - 2.0 % by weight of sweetener
g) 0.1 - 5.0 % by weight of aroma
h) 0.1- 10.0 % by weight of sodium stearyl fumarate
i) 3.0 - 5.0 % by weight of coating

12. The orally disintegrating formulation-according to any preceding claim comprising;
a) 2.0 - 80.0 % by weight of linagliptin
b) 0.1 - 50.0 % by weight of sodium starch glycolate
c) 0.5 - 90.0 % by weight of microcrystalline cellulose
d) 0.1 - 1.0 % by weight of silica
e) 0.1 - 2.0 % by weight of sweetener
f) 0.1 - 5.0 % by weight of aroma
g) 0.1 - 10.0 % by weight of sodium stearyl fumarate
h) 5.0 - 60.0 % by weight of sodium bicarbonate
i) 5.0 - 60.0 % by weight of citric acid anhydrate
j) 2.5 - 5.5 % by weight of xylitol
k) 0.2 - 1.0 % by weight of sucrose stearate
l) 3.0 - 5.0 % by weight of coating
